# EUROPEAN PATENT SPECIFICATION

(11) **EP 2 941 227 B1**
(45) Date of publication and mention of the grant of the patent: **02.08.2017**
(21) Application number: 13818225.8
(22) Date of filing: 27.12.2013
(51) Int. Cl.: A61F 5/055

(54) **CERVICAL BRACE**
CERVICALSTÜTZE
MINERVE

(30) Priority: 28.12.2012 SE 1200795
(43) Date of publication of application: 11.11.2015
(73) Proprietor: Cervrite Ab, 44163 Alingsås (SE)
(72) Inventor: ROSENFELD, Mark, 441 63 Alingsås (SE); MANGOLD, Stephan, 431 38 Mölndal (SE); MOZER, Dieter, 507 33 Brämhult (SE)
(74) Representative: Valea AB
(86) International application number: PCT/EP2013/078069
(87) International publication number: WO 2014/102340

(56) References cited:
- WO-A1-2009/096895
- US-A- 2 820 455

## Description

### TECHNICAL FIELD

The present invention concerns an easily applied and fitted rigid cervical brace providing comfort, stability and immobility to the neck and head in the neutral position. It prevents movement in all directions but allows controlled rotation of the head and neck, only when prescribed and electronically monitored, to prevent further injury and enhance the management, healing and rehabilitation of neck disorders and traumatic neck injuries, such as whiplash injuries, with or without neurological signs and symptoms.

### BACKGROUND OF THE INVENTION

There is a consensus for the need of initial or prolonged immobilisation for a number of neck disorders including traumatic neck injuries. There is also evidence for the effectiveness of early neck movement (rotation) in a doctoral thesis (Rosenfeld M. (2006) Whiplash-associated disorders from a physical therapy and health-economic perspective. Göteborg University, Göteborg) which has shown that healing and rehabilitation of neck injuries in motor vehicle crashes were significantly improved, and costs for these injuries significantly reduced, if patients soon after injury or early in the process of illness, regularly exercised the neck through repeated rotation exercises. This thesis confirms previous studies showing the benefits of early movement in the treatment of neck and other musculoskeletal injuries. On the other hand, initial but limited immobilisation protects the neck from further damage when there is extensive injury to the neck or insufficient stability, while early initiated neck rotation addresses problems caused by damaged nerves, joints and soft tissue by mobilizing these structures, when tolerated, to prevent scar tissue from forming adhesions causing later dysfunction, as well as by keeping neck muscles fit by preventing muscle atrophy, weakness and contraction. Movement also provides valuable stimulation of proprioception (movement feedback), essential for balance and coordination. Neck rotation also avoids the longitudinal stress on the neural structures caused by excessive head flexion/extension (forward and backward head bending) and lateral flexion (side bending) of the head and neck. Mobilisation also reduces possible inhibition of blood flow due to compression. Animal studies have shown the negative effects of prolonged immobilization on joints, and that cartilaginous, articular repair is significantly enhanced by movement and loading and is an important stimulus to joint regeneration processes. Early movement also addresses the issue of fear of movement and avoidance of activity, which together, can contribute to delayed recovery. Rotation exercises have thus become an accepted part of the treatment of acute whiplash injuries throughout the world. There is also a potential benefit to other conditions requiring initial stability followed by mobilisation through active movement.

A distinction, however, must be made at this point to differentiate larger or excessive movements of the neck as mentioned earlier (lateral flexion or side bending of the head and neck in the frontal plane, and flexion-extension, back and forth movements of the head and neck, in the sagittal plane) which can damage traumatised tissue, and the small non-injurious combined movements that occur in limited, controlled rotation of the neck necessary for healing and recovery of the neck.

There is, at present, no orthopaedic, technical device that allows beneficial healing and rehabilitating, anatomically and biomechanically correct rotational movements of the head and neck while simultaneously providing stability and support that is so user-friendly that the user can independently put on or remove the brace.

Patent application US2820455 discloses a neck brace which is adjustable to hold the head of the wearer in any one of a number of positions. The brace can be adapted to hold the head in a predetermined canted or tilted position for a certain length of time, and later to hold the head in a second canted or tilted position as may be required by the particular deformity or fracture being corrected or healed. However, this neck brace will not allow the head and neck to rotate, as it fixes the head in one position at the time and the patient cannot move the head freely.

US 2008/004556 A1 discloses a cervical brace allowing varying mobility (rotation) to the cervical region with the range of motion or degree of mobility being adjustable and quantifiable. A chin support is constructed so it can be slidably displaced in an arc so that the patient whose chin is supported by the chin support can correspondingly rotate the head. A major limitation of this brace is that rotation occurs in only one (transverse or horizontal) plane as determined by an arc described by the chin support. This one-dimensional rotation follows neither the anatomy of the neck nor the biomechanical movement of the neck joints. Another cervical brace in accordance with the preamble of claim 1 is disclosed in WO 2009/096895. The cervical brace of the invention, on the other hand has been specially designed and constructed to correspond biomechanically and anatomically to the unique morphology determining the dynamic movements of the cervical spine in prescribed anatomical rotation while inhibiting other unwanted larger movements as described above. It has a chin support wherein the chin is fixated, which is freely rotatable in three planes adjusting for variations in the axes and planes of rotation exhibited during axial rotation of the head.

### SUMMARY OF THE INVENTION

The present invention relates to a cervical brace providing support and stability to the neck of a wearer arranged to prevent undesired excessive cervical flexion, extension and lateral flexion while allowing desired and controlled discrete cervical rotation. The cervical brace comprises a shoulder brace arranged to rest on the shoulders of the wearer. The shoulder brace is made from a stiff, yet slightly bendable material in order to be able to adjust the brace to fit the shoulders of the individual wearer.

The shoulder brace supports a head piece configured to encircle and hold the back of the neck of the wearer. It extends from about the first ear, behind the head to the second ear. The head piece is connected to the shoulder brace by at least two supportive connecting means, one on each shoulder brace, which preferably are adjustable in the lengthwise direction. However, additional supportive connecting means may be attached if increased stability is required. Both the head piece and the connecting means are made from a rigid and stiff material to provide stability to the brace. The head piece contains an adjustable attached headrest to support the back of the head of the wearer. The head rest may be raised or lowered to improve the fit and comfort of the brace for the individual wearer.

A front support member arranged with a chin support movably mounted thereon is attached to the head piece. Said front support member is fastened to each end of the head piece, and forms a half circle in front of the face at the level of the chin of the wearer. The wearer's chin rests on the chin support which is slidably arranged onto a movable rotation member. The movable rotation member is freely rotatable in three planes of three axes of rotation to allow cervical rotation while preventing excessive cervical flexion, extension and lateral flexion of the neck.

As used herein, the expression, "freely rotatable in three planes of three axes of rotation" is intended to mean that the movable rotation member with the chin support is rotatable in an indefinite number of planes of movement. For the purpose of the present invention rotation may occur in three planes concurrently and may be symbolized by simultaneous and combined, or linked axial rotation of the head around the vertical (i.e. the longitudinal) axis in the horizontal plane, combined with extension movement on a frontal axis in the sagittal plane, and lateral flexion (side bending) around the sagittal axis in the frontal (coronal) plane, with respect to a wearer of the cervical brace. Axial rotation around the vertical axis, (longitudinal) i.e. the axis which runs straight down through the top of the head, means rotation of the head from one side to the other and is combined with extension and lateral flexion of the neck as determined by the anatomy of the cervical spine as described below.

A major limitation of prior art braces is that they either allow no rotation whatsoever or they allow rotation only in one (horizontal) plane, i.e. rotation around the vertical axis, as determined by an arc described by the chin support. This one-dimensional rotation follows neither the anatomy of the neck nor the biomechanical movements of the neck joints.

Anatomically the units of the cervical spine include the atlas, the axis, the C2-3 junction and the remaining typical cervical vertebrae. A large range of axial rotation of the head occurs at the atlanto-axial joint where the articular cartilages both of the atlantial and the axial facets of the joint are convex, rendering the joint biconvex. As the atlas rotates, the ipsilateral atlantial facet slides down the posterior slope of its axial facet, and the contralateral atlantial facet slides down the anterior slope of its facet. As a result, during axial rotation of the head the atlas descends, or nestles into the axis and upon reversing the rotation, the atlas rises back onto the summits of the facets. Therefore the atlanto-occipital joint is an enarthrosis, i.e., a joint with spherical articular surfaces with three degrees of freedom: axial rotation about a vertical (longitudinal) axis, flexion on a frontal axis, and lateral flexion on a sagittal axis.

Therefore, rotation of the occiput to the left is associated at once with a linear displacement and lateral flexion to the right and vice versa. Further, due to the oblique orientation of the facet joints of the lower cervical spine (below C2) lateral flexion in connection with rotation becomes further accentuated so that rotation at each segment of the cervical column occurs on an oblique axis and is always associated with lateral flexion and that the cervical vertebral column can only perform movements of mixed lateral flexion-rotation-extension. Thus, if rotation is limited to one plane, rotation will be resisted. This can be significant not only for uninjured subjects attempting to carry out rotation but especially for a patient with an injured cervical spine.

The cervical brace of the invention on the other hand has been specially constructed to correspond biomechanically and anatomically to the unique morphology determining the dynamic movement of the cervical spine. It has been designed to adjust for variations in the axes and planes of rotation by means of a movable rotation member with a chin support wherein the chin is fixated that is "freely rotatable in three planes of three axes of rotation". The rotational movement of the movable rotation member with the chin support is concurrent in three planes simultaneously and is substantially frictionless in order to not restrict the natural movement pattern of the head and spine during the rotation of the head. It is essential that the brace does not induce any external restraints on the neck or cause any displacement to the vertebrae in an already weakened neck.

The front support member may be a rod-shaped fixture with an undulating shape with at least one upper turning point. A rotational support is fixed to the front support member on at least one upper turning point. The front support member is adjustably attached to the head support to regulate the distance between the neck support and the upper turning point on the front support member such that the upper turning point with the rotational support coincides with the wearer's chin.

The rotational support comprises a rotational support connector which fixes the rotational support to the front support member, and the movable rotation member which allows rotational movement of the chin support which is slidably arranged thereto.

The chin support is slidably coupled to the rotational support through a sliding arrangement, enabling a concurrent rotational movement of the chin support in a horizontal, sagittal, and frontal plane on the rotational support. Advantageously said sliding arrangement is a sliding means such as a sled that slides in a slider receiving means, such as a runner arranged on the rotational support. The sliding motion is performed in a substantially frictionless manner.

The movable rotation member is connected to the rotational support connector, which enables rotation of the movable rotation member with respect to the rotational support connector, which is arranged in a fixed position to the front support. Said movable rotational means has a vertical axis of rotation and enables the chin support which is fixed onto said movable rotation member to rotate on said vertical axis in the horizontal plane of rotation with respect to the wearer. Advantageously a rotational movement of 20° or less, is allowed in either direction around the vertical axis of the movable rotation member.

The movable rotational means has a sagittal axis of rotation and enables the chin support, which is mounted onto said movable rotation member to rotate on said sagittal axis in the frontal plane of rotation with respect to the wearer allowing discrete lateral flexion movements of the head (i.e. moving of the ears towards the shoulders). Advantageously, a maximal rotational movement of 10° is allowed in either direction on a sagittal axis of the movable rotation member.

The movable rotational means has a frontal axis of rotation and enables the chin support, which is mounted onto said movable rotation member to rotate on said frontal axis in the sagittal plane of rotation with respect to the wearer, allowing discrete flexion and extension movement (i.e. movement of the chin towards the chest and back). Advantageously, a maximal rotational movement of 10° is allowed in either direction on the sagittal axis of the movable rotation member.

The front support member is bent circularly in front of the face of the wearer and is adjustably attached to the neck support on each side of the head. It is adjustable in the horizontal as well as the vertical direction to regulate the distance between the neck support and the upper turning point on the front support member such that the upper turning point with the rotational support coincides with the wearer's chin. Thus, by means of the movable rotation member and the sliding arrangement mounted thereon the rotatable movement of the chin support is enabled in all three planes, i.e. the horizontal plane, the frontal plane and the sagittal plane.

The sliding motion of the sliding means with the chin support may be restricted by adjustable regulating means, such as stop screws. Restriction of head movement may be necessary in the early stages of the healing process or in the case of instability due to fracture or ligamentous insufficiency. Advantageously varying degrees of rotation can be allowed depending on the degree of healing, tissue and patient tolerance. There is, thus a possibility to determine the degree of rotation from a few degrees to maximum degrees by adjusting and locking the sliding motion of the sled on the sliding support. The sliding motion of the chin support may also be completely restricted.

The cervical brace of the invention may further comprise an electronic monitoring device arranged to register sliding motions of the chin support. To register compliance by monitoring the number of rotation exercises performed, the device may be furnished with an electronic monitoring device. The regulating means, such as e.g. the stop screws may be equipped with micro current circuit breakers, magnetic reed switches and a magnet. The micro current circuit breakers or magnetic reed switches are connected to a low power, single chip computer such as for example the PIC12C50, and a real time clock such as for example the DS1302, powered by a single cell battery. At a follow-up appointment, data on how often and when the head has been rotated throughout the healing process can be gathered from the electronic monitoring device via a connector, which is connected to the physician's computer.

The digital transfer between the electronic monitoring device and the computer can be wireless made, optically with infrared waves (IR) or with radio waves (Bluetooth). The electronic monitoring device can also send an alarm signal to the user's cell phone, personal digital assistant (PDA) or computer to remind them to perform the rotation exercise.

### BRIEF DESCRIPTION OF THE DRAWINGS

Figure 1 is a schematic view of the cervical brace of the invention.
Figure 2 is a view of how the front support member is fitted to the head support.
Figure 3 discloses schematically how the chin support can rotate in three dimensional planes.
Figure 4 shows a cross sectional view of the chin support
Figure 5 shows the movements of the chin support as viewed from above during rotation of the head in the x-y plane, wherein A) the chin support slides on the sliding support, B) the head rotates to the left, and C) the head rotates to the right.
Figure 6 shows the movements of the chin support as viewed from the front during lateral flexion of the head in the x-z plane, wherein A) the head does not move, but faces the front, B) the head flexes towards the left, and C) the head flexes towards the right.
Figure 7 shows the movements of the chin support as viewed from the side during extension of the head in the y-z plane, wherein A) the head does not move, but faces the front, B) the head bends forward, and C) the head extends backward.
Figure 8 is a perspective side view of a patient fitted with the cervical brace of the invention - no rotation of the head.
Figure 9 is a perspective side view of a patient fitted with the cervical brace of the invention during rotation of the head to the left.

### DETAILED DESCRIPTION OF PREFERRED EMBODIMENTS

In the following the cervical brace of the invention will be described in a non-limiting way and in more detail with reference to exemplary embodiments illustrated in the enclosed drawings. However, the described embodiments mentioned below are only given as examples and should not be limiting to the present invention. Other solutions, uses, objectives, and functions within the scope of the invention as claimed in the below described patent claims should be apparent for the person skilled in the art. It should be noted that the word "comprising" does not exclude the presence of other elements or steps than those listed and the words "a" or "an" preceding an element do not exclude the presence of a plurality of such elements.

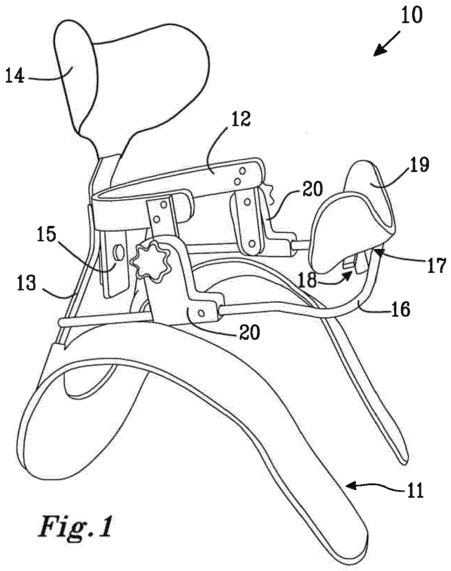

Figure 1 shows the cervical brace 10 of the invention, said cervical brace comprising a shoulder brace 11 intended to rest on the shoulders of the wearer. The shoulder brace 11 is made from a stiff and rigid material. Preferably the shoulder brace 11 is made from a light weight, yet somewhat bendable material, such as aluminum. It is advantageous that the brace is slightly malleable in order to enable adjustment of the fit to the individual wearer. The shoulder brace 11 may also be made from an adaptable plastic material, such as polypropylene.

A head piece 12 is fitted to the shoulder brace 11. The head piece 12 is designed to encircle the back of the head of the wearer, reaching from around the first ear, behind the head to the second ear. The head piece 12 is connected to the shoulder brace 11 by at least two supportive connecting means 13, one on each shoulder brace 11, which preferably are adjustable in the lengthwise direction. However, additional supportive connecting means 13 may be added in case increased stability is required. Both the head piece 12 and the connecting means 13 are made from a rigid and stiff material to provide stability to the brace. The head piece 12 contains a head rest 14 that is adjustably attached to the head piece 12 to support the back of the head of the wearer. The head rest 14 may be adjusted in the vertical direction by means of a head rest adjustment means 15, i.e. it can be raised or lowered to improve the fit of the brace to the individual wearer. The head rest 14 is advantageously covered with a soft material to enhance the comfort when the wearer rests the back of his head against the head rest 14.

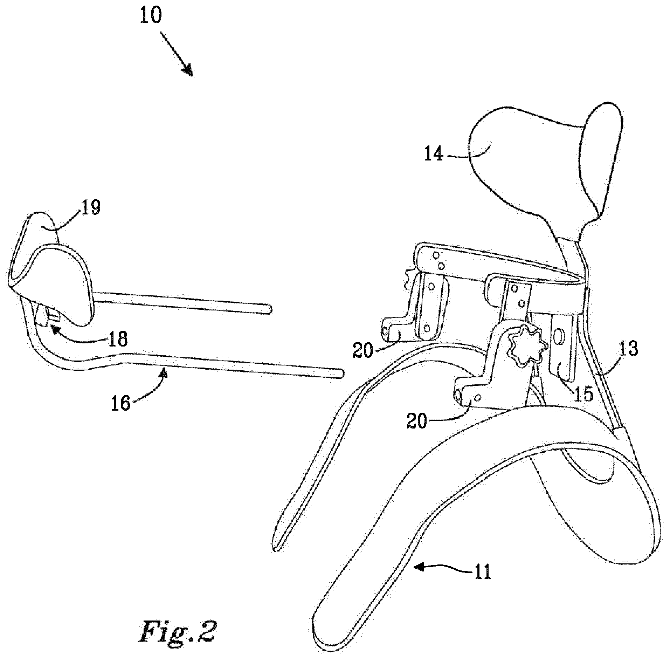

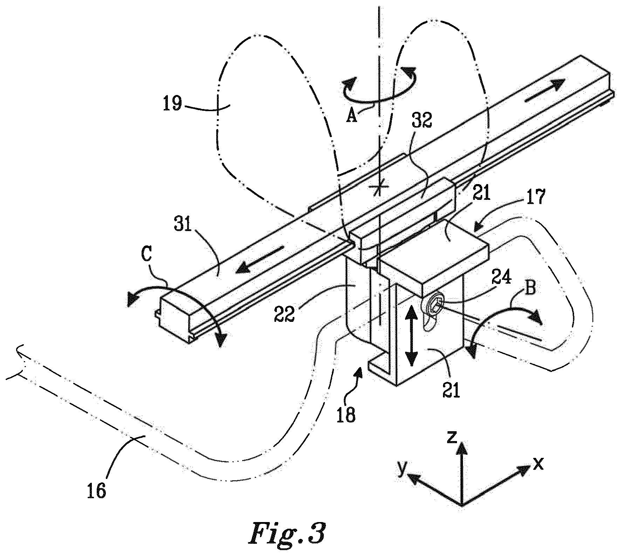

The head piece 12 supports a front support member 16 that encircles the face in front of the chin of the wearer. The front support member 16 can be released completely from the head piece 12 to facilitate fitting of the cervical brace 10 to the wearer (see figure 2) Said front support member 16 has an undulating shape with at least one upper turning point 17, as can be seen in Figure 3. A rotational support 18 onto which a chin support 19 is slidably arranged, is mounted onto the front support member 16 at the upper turning point 17. Advantageously the front support member 16 is a rigid rod-shaped fixture made from a metal material. The front support member 16 is fastened to the head piece 12 by head piece connectors 20 (see Figure 2). Head piece connectors 20 arranged at each end of the head piece 12 enable adjustment of the front support member 16 in the horizontal direction, as well as in the vertical direction, to regulate the distance between the head piece 12 and the upper turning point 17.

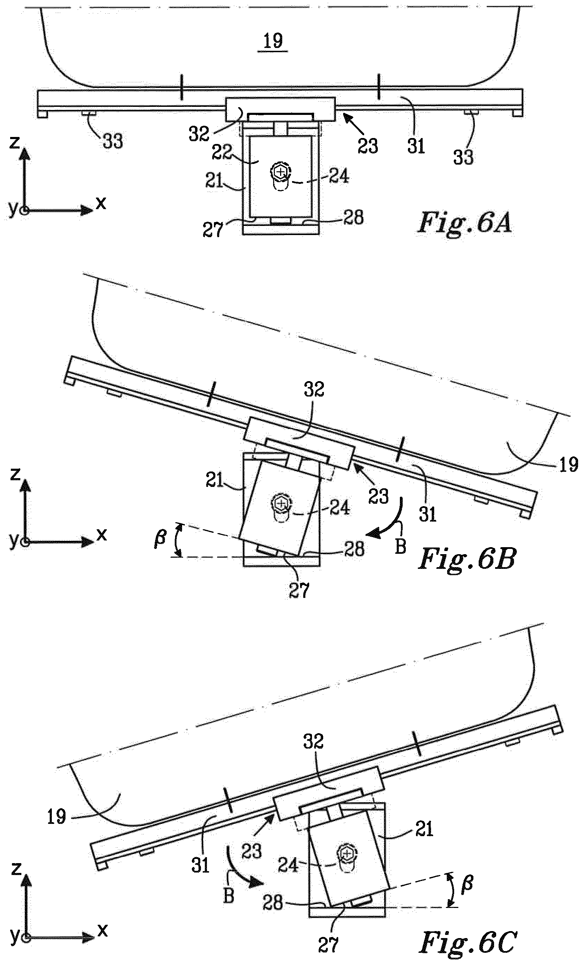

The rotational support 18 is arranged at the upper turning point 17 of the front support member 16. The rotational support 18 comprises a rotational support connector 21 with a movable rotation member 22 arranged thereto. Said rotational support connector 21 connects the rotational support 18 to the front support member16, and said movable rotation member 22 which enables rotational movement of the chin support 19 which is slidably arranged thereto via a sliding arrangement 23. The movable rotation member 22 is fitted adjacent to and/or inside the rotational support connector 21 and connected thereto by means of a screw 24. The screw 24 enables rotation of the movable rotation member 22 together with the chin support 19 around the sagittal axis, i.e. in the frontal plane, with respect to the wearer of the cervical brace (see figure 6). The rotational support 18 is adjustable in the vertical direction to enable improved fit of the chin support 19.

However, as pointed out above, although rotation of the head involves a simultaneous rotational movement of the head in all three planes, the movement in at least two of the planes, the frontal plane and the sagittal plane, is very limited. Therefore, the rotational movement of the movable rotation member 22 is restricted to only a few degrees in these two planes of rotation.

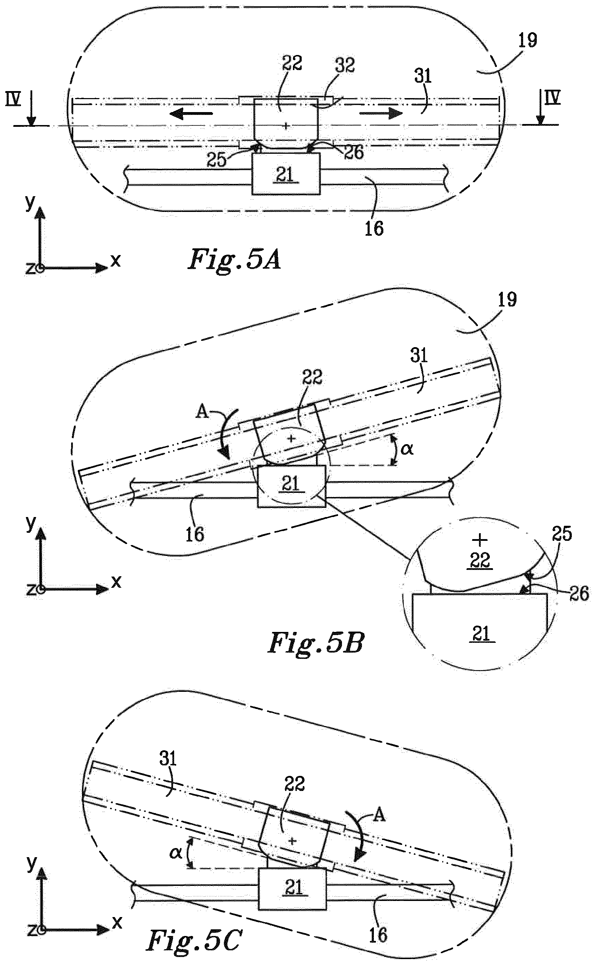

The motion of the movable rotation member 22 in the horizontal plane is shown by a rotational arrow A in figures 3, 5B and 5C which illustrates the rotational movement of the movable rotation member 22 around its vertical axis, (which is collinear with the vertical axis of the wearer when the cervical brace is fitted to the wearer), from above. The front side of the movable rotation member 22 (i.e. the side facing the rotational support connector 21) is provided with a slightly convex surface 25 in the lateral direction (see insert in figure 5). Thus, when the movable rotation member 22 rotates around its vertical axis it will rotate until the convex surface 25 abuts a first abutment surface 26 on the rotational support connector 21. Depending on the curvature of the convex surface 25, different degrees of rotation may be allowed. Figure 5B illustrates the movement of the movable rotation member 22 together with the chin support 19 during rotation of the head to the left. Figure 5C illustrates the movement of the movable rotation member 22 together with the chin support 19 during rotation of the head to the right. Preferably, a rotational movement of 20° or less as illustrated by α in the figures 5B and C is allowed in either direction. However, depending on the status of the patient the rotation around the vertical axis may be completely restricted, or restricted to only a few degrees in either direction around the vertical axis of the movable rotation member 22.

The restricted motion of the movable rotation member 22 in the frontal plane is shown by a rotational arrow B in figures 3, 6B and 6C which illustrates the rotational movement of the movable rotation member 22 around its sagittal axis, (which is collinear with the sagittal axis when the cervical brace is fitted to the wearer), as viewed from the front. The movable rotation member 22 is provided with a bottom surface 27 which faces a second abutment surface 28 on the rotational support connector 21. Thus, when the movable rotation member 22 rotates around its sagittal axis it will rotate until the bottom surface 27 abuts the second abutment surface 28 on the rotational support connector 21. Thus, depending on the distance between the bottom surface 27 and the second abutment surface, different degrees of rotation may be allowed. Figure 6B illustrates tilting of the head to the left (i.e. movement of left ear towards the left shoulder) and figure 6C illustrates tilting of the head to the right (i.e. movement of right ear towards the right shoulder). Preferably, a maximal rotational movement of 10°, as illustrated by β in figures 6B and C, is allowed in either direction around the sagittal axis of the movable rotation member 22.

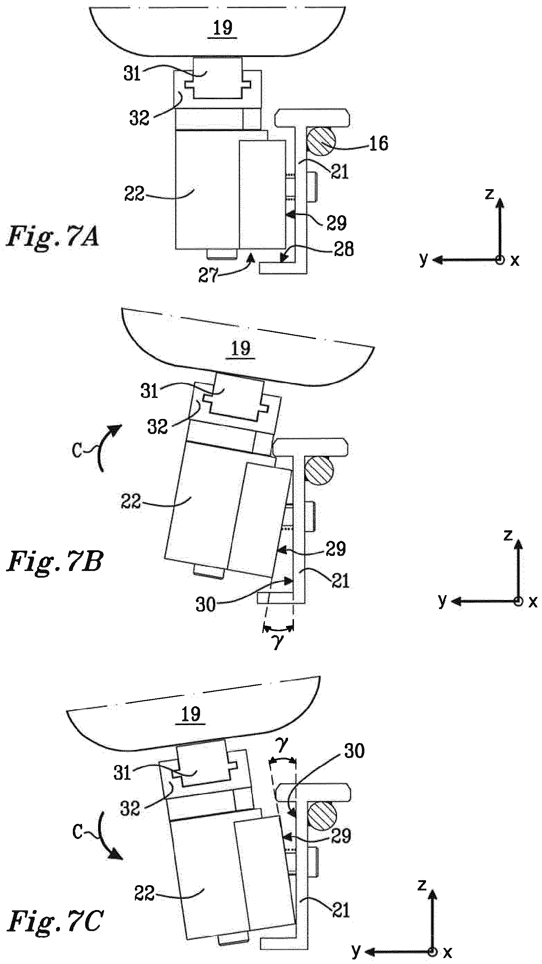

The restricted motion of the movable rotation member 22 in the sagittal plane is shown by a rotational arrow C in figures 3, 7B and 7C which illustrates the rotational movement of the movable rotation member 22 around its frontal axis, (which is collinear with the frontal axis of the wearer of the brace), as viewed from the side. The movable rotation member 22 is provided with a front surface 29 which faces a third abutment surface 30 on the rotational support connector 21. Thus, when the movable rotation member 22 rotates around its frontal axis it will rotate until the front surface 29 abuts the third abutment surface 30 on the rotational support connector 21. Thus, depending on the distance of the front surface 29 to the third abutment surface 30, different degrees of flexion/extension may be allowed. Figure 7B illustrates forward bending (flexion) and figure 7C illustrates backward bending (extension). Preferably, a maximal movement of 10° (as illustrated by γ in figures 7B and C) is allowed in either direction around the frontal axis of the movable rotation member 22.

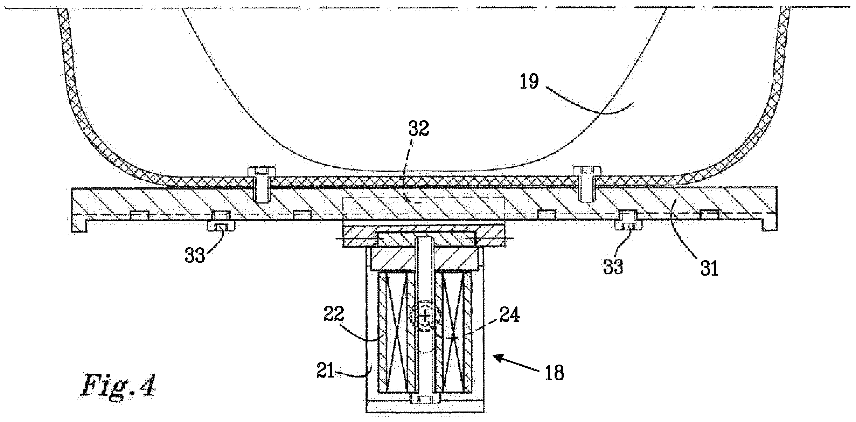

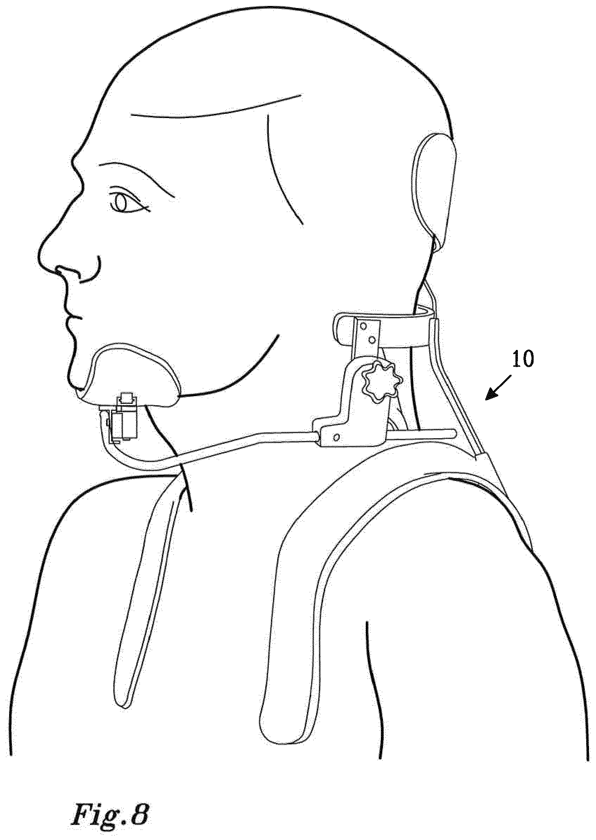

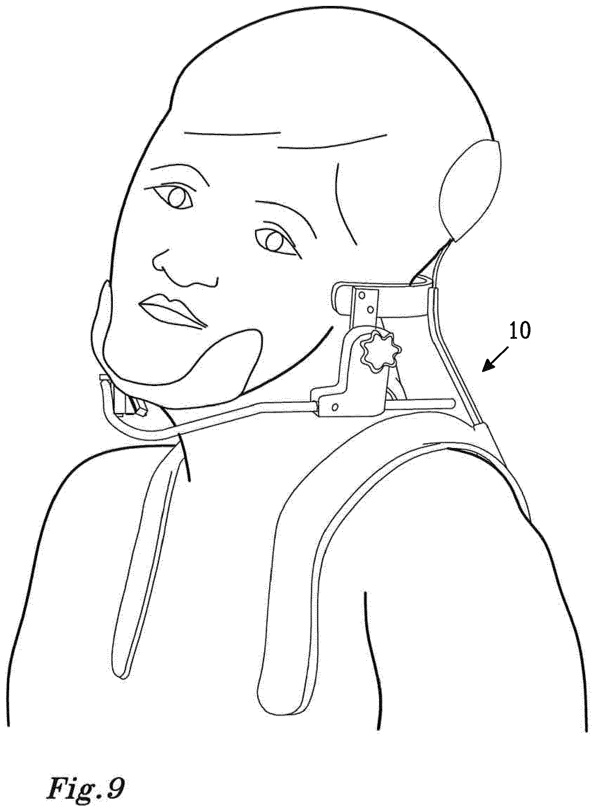

The chin support 19 is slidably coupled to the rotational support 18 via a sliding arrangement 23. Said sliding arrangement 23 comprises a sliding means 31, such as e.g. a sled that will slide in a virtually frictionless manner in a slider receiving means 32, such as a runner. The sliding arrangement 23 enables movement of the chin support 19 which is fixed to the sliding means 31, back and forth along the slider receiving means 32 as can be seen in figures 4 -7. The slider receiving means 32 is fixed to an upper side of the movable rotation member 22 and will therefore rotate with the movable rotation member 22, to the right and left, tilt from side to side and back and forth, as enabled by the movable rotation member 22. Depending on the direction and inclination of the slider receiving means 32, the direction of the slidable movement of the sliding means 31 together with the chin support 19 will take on the same direction and inclination as specified by the slider receiving means 32. Thus, by means of the movable rotation member 22 and the sliding arrangement 23 mounted thereon the rotational movement of the chin support 19 is enabled in all three planes, i.e. the horizontal plane, the frontal plane and the sagittal plane concurrently. The rotational movement of the head as enabled by the cervical brace of the invention is illustrated in figures 8 and 9, wherein a side view of a wearer looking straight ahead can be seen in figure 8, and after rotation of the head to the left in figure 9. It can be seen that the cervical brace of the invention enables axial rotation on a vertical axis, flexion and rotation on a frontal axis, and lateral flexion on a sagittal axis, with respect to the wearer.

The sliding motion of the sliding means 31 with the chin support 19 may be restricted by adjustable regulating means 33, such as stop screws. The adjustable regulating means 33 are positioned along the sliding means 31 so as to restrict the sliding motion of the sliding means 31 along the slider receiving means 32. The adjustable regulating means 33 are advantageously movable along the sliding means 31, so as to restrict the movement from no movement at all to full movement along the entire length of the sliding means 31.

The adjustable regulating means, such as e.g. the stop screws may be equipped with micro current circuit breakers, magnetic reed switches and a magnet (not shown). The micro current circuit breakers or magnetic reed switches are connected to a low power, single chip computer such as for example the PIC12C50, and a real time clock such as for example the DS1302, powered by a single cell battery. The computer, battery and accessories are mounted in a small electronic unit fastened, for example, inside the lower rear part of the head piece 12 (not shown). At a follow-up appointment, data can be gathered from the electronic monitoring device via a connector, which is connected to the physician's computer, for example, via a USB port. It is then possible to see how the patient has utilized the cervical brace by seeing, for example, how often and when the head has been rotated throughout the healing process. The digital transfer between the electronic monitoring device and the computer can be wireless made, optically with infrared waves (IR) or with radio waves (Bluetooth). If a wireless transmission is used, such as Bluetooth, the information can be transferred to the user's cell phone, personal digital assistant (PDA) or computer in order to notify the user how many rotations have been made during the day. With this information the user can increase the number of rotations if the user has not reach the prescribed number for the day. The electronic monitoring device can also send an alarm signal to the user's cell phone, personal digital assistant (PDA) or computer to remind them to perform the rotation exercise. The alarm signal can also be installed in the software of the user's cell phone, personal digital assistant (PDA) or computer. The electronic unit can also be equipped with a sounding alarm, such as a small loudspeaker, producing an alarming signal, for example hourly, when the user is to perform the rotation exercise. The physician can pre-program or reprogram the intervals between alarms

The above mentioned and described embodiments are only given as examples and should not be limiting to the present invention. Other solutions, uses, objectives, and functions within the scope of the invention as claimed in the below described patent claims should be apparent for the person skilled in the art.

## Claims

1. A cervical brace (10) providing support and stability to the neck of a wearer, said cervical brace comprising
a shoulder brace (11) arranged to rest on the shoulders of the wearer; and
a head piece (12) supported by the shoulder brace (11) configured to encircle the back of the neck of the wearer; and
a head rest (14) adjustably attached to the head piece (12) to support the back of the head of the wearer; and
a front support member (16) arranged with a chin support (19) movably mounted thereon is attached to the head piece (12),
**characterized in that** said chin support (19) is slidably arranged onto a movable rotation member (22) which is freely rotatable in three planes of three axes of rotation to allow cervical rotation while preventing excessive cervical flexion, extension and lateral flexion of the neck.

2. The cervical brace according to claim 1, **characterized in that** the movable rotation member (22) is rotatable in a horizontal, sagittal, and frontal plane.

3. The cervical brace according to claims 1 and 2, **characterized in that** the front support member (16) has an undulating shape with at least one upper turning point (17).

4. The cervical brace according to claim 3, **characterized in that** a rotational support (18) is fixed to the front support member (16) at the at least one upper turning point (17).

5. The cervical brace according to claim 4, **characterized in that** the rotational support (18) comprises a rotational support connector (21) with the movable rotation member (22) arranged thereto, said rotational support connector (21) connects the rotational support (18) to the front support member (16).

6. The cervical brace according to claim 5, **characterized in that** the chin support (19) is slidably coupled to the movable rotation member (22) by means of a sliding arrangement (23) enabling a concurrent rotational movement of the chin support (19) in a horizontal, sagittal, and frontal plane onto the rotational support (18).

7. The cervical brace according to claim 6, **characterized in that** the sliding arrangement (23) comprises a sliding means (31) that slides in a slider receiving means (32) in a substantially frictionless manner.

8. The cervical brace according to any one of the preceding claims 5-7, **characterized in that** the movable rotation member (22) is connected to the rotational support connector (21) by means of a screw.

9. The cervical brace according to any one of the preceding claims 5-8, **characterized in that** the movable rotational member (22) has a vertical axis of rotation and enables the chin support (19) to rotate in a rotational movement of 20° or less in either direction around said vertical axis in the horizontal plane of rotation with respect to the wearer.

10. The cervical brace according to any one of the preceding claims 5-9, **characterized in that** the movable rotational member (22) has a frontal axis and enables the chin support (19) to rotate in a maximal rotational movement of 10° in either direction around said frontal axis in the sagittal plane of rotation with respect to the wearer.

11. The cervical brace according to any one of the preceding claims 5-10, **characterized in that** the movable rotational member (22) has a sagittal axis and enables the chin support (19) to rotate in a maximal rotational movement of 10° in either direction around said sagittal axis in the frontal plane of rotation with respect to the wearer.

12. The cervical brace according to any one of the preceding claims 3-11, **characterized in that** the front support member (16) is adjustably attached to the head piece (12) to regulate the distance between the head piece (12) and the upper turning point (17) on the front support member (16) such that the upper turning point (17) with the rotational support (18) coincides with the wearer's chin.

13. The cervical brace according to any one of the preceding claims 6-12, **characterized in that** the sliding motion of the sliding means (31) with the chin support (19) is restricted by adjustable regulating means (33).

14. The cervical brace according to claim 13, **characterized in that** the sliding motion of the sliding means (31) with the chin support (19) is completely restricted.

15. The cervical brace according to any one of the preceding claims 1-14, **characterized in that** the brace further comprises an electronic monitoring device arranged to register, monitor and store information about sliding motions of the chin support (19).

## Patentansprüche

1. Eine Halsstütze (10), die dem Hals von einem Träger Abstützung und Stabilität bereitstellt, die besagte Halsstütze umfassend:
eine Schulterstütze (11), die angeordnet ist, um auf den Schultern von dem Träger zu liegen; und
ein Kopfteil (12), das von der Schulterstütze (11) abgestützt und konfiguriert ist, um die Hinterseite von dem Hals von dem Träger zu umgeben; und
eine Kopfauflage (14), die einstellbar an dem Kopfteil (12) befestigt ist, um den Hinterkopf von dem Träger abzustützen; und
ein vorderes Stützelement (16), das mit einer beweglich darauf montierten Kinnstütze (19) angeordnet ist, ist an dem Kopfteil (12) befestigt,
**dadurch gekennzeichnet, dass** die besagte Kinnstütze (19) auf einem beweglichen Drehelement (22) verschiebbar angeordnet ist, das in drei Ebenen von drei Drehachsen frei drehbar ist, um eine Halsdrehung zu erlauben und gleichzeitig eine übermäßige Halsflexion, Streckung und Querflexion von dem Hals zu verhindern.

2. Die Halsstütze nach Anspruch 1, **dadurch gekennzeichnet, dass** das bewegliche Drehelement (22) in einer Horizontal-, Sagittal- und Frontalebene drehbar ist.

3. Die Halsstütze nach Ansprüchen 1 und 2, **dadurch gekennzeichnet, dass** das vordere Stützelement (16) eine wellenförmige Gestalt mit wenigstens einem oberen Wendepunkt (17) hat.

4. Die Halsstütze nach Anspruch 3, **dadurch gekennzeichnet, dass** eine Drehstütze (18) an dem vorderen Stützelement (16) an dem wenigstens einen oberen Wendepunkt (17) befestigt ist.

5. Die Halsstütze nach Anspruch 4, **dadurch gekennzeichnet, dass** die Drehstütze (18) einen Drehstützverbinder (21) mit dem daran angeordneten beweglichen Drehelement (22) umfasst, wobei der besagte Drehstützverbinder (21) die Drehstütze (18) mit dem vorderen Stützelement (16) verbindet.

6. Die Halsstütze nach Anspruch 5, **dadurch gekennzeichnet, dass** die Kinnstütze (19) mit dem beweglichen Drehelement (22) mittels einer Schiebeanordnung (23) drehbar gekoppelt ist, wodurch eine gleichzeitige Drehbewegung von der Kinnstütze (19) in einer Horizontal-, Sagittal- und Frontalebene auf der Drehstütze (18) ermöglicht ist.

7. Die Halsstütze nach Anspruch 6, **dadurch gekennzeichnet, dass** die Schiebeanordnung (23) ein Schiebemittel (31) umfasst, das in einem Schieberempfangsmittel (32) auf eine im Wesentlichen reibungsfreie Art und Weise gleitet.

8. Die Halsstütze nach einem der vorangegangenen Ansprüche 5-7, **dadurch gekennzeichnet, dass** das bewegliche Drehelement (22) mittels einer Schraube mit dem Drehstützverbinder (21) verbunden ist.

9. Die Halsstütze nach einem der vorangegangenen Ansprüche 5-8, **dadurch gekennzeichnet, dass** das bewegliche Drehelement (22) eine vertikale Drehachse hat und ermöglicht, dass sich die Kinnstütze (19) in einer Drehbewegung von 20° oder weniger in beide Richtungen um die besagte vertikale Achse in der horizontalen Drehebene bezogen auf den Träger dreht.

10. Die Halsstütze nach einem der vorangegangenen Ansprüche 5-9, **dadurch gekennzeichnet, dass** das bewegliche Drehelement (22) eine Frontalachse hat und ermöglicht, dass sich die Kinnstütze (19) in einer maximalen Drehbewegung von 10° in beide Richtungen um die besagte Frontalachse in der Sagittaldrehebene bezogen auf den Träger dreht.

11. Die Halsstütze nach einem der vorangegangenen Ansprüche 5-10, **dadurch gekennzeichnet, dass** das bewegliche Drehelement (22) eine Sagittalachse hat und ermöglicht, dass sich die Kinnstütze (19) in einer maximalen Drehbewegung von 10° in beide Richtungen um die besagte Sagittalachse in der Frontaldrehebene bezogen auf den Träger dreht.

12. Die Halsstütze nach einem der vorangegangenen Ansprüche 3-11, **dadurch gekennzeichnet, dass** das vordere Stützelement (16) einstellbar an dem Kopfteil (12) befestigt ist, um den Abstand zwischen dem Kopfteil (12) und dem oberen Wendepunkt (17) auf dem vorderen Stützelement (16) zu regeln, so dass der obere Wendepunkt (17) mit der Drehstütze (18) mit dem Kinn des Trägers zusammenfällt.

13. Die Halsstütze nach einem der vorangegangenen Ansprüche 6-12, **dadurch gekennzeichnet, dass** die Gleitbewegung von dem Schiebmittel (31) mit der Kinnstütze (19) durch einstellbare Regelmittel (33) beschränkt ist.

14. Die Halsstütze nach Anspruch 13, **dadurch gekennzeichnet, dass** die Gleitbewegung von dem Schiebemittel (31) mit der Kinnstütze (19) vollständig beschränkt ist.

15. Die Halsstütze nach einem der vorangegangenen Ansprüche 1-14, **dadurch gekennzeichnet, dass** die Stütze weiter eine elektronische Überwachungsvorrichtung umfasst, die angeordnet ist, um Informationen über Gleitbewegungen von der Kinnstütze (19) zu registrieren, zu überwachen und zu speichern.

## Revendications

1. Minerve (10) fournissant un soutien et une stabilité au cou d'un porteur, ladite minerve comprenant
une épaulière (11) agencée pour reposer sur les épaules du porteur ; et
une pièce de tête (12) supportée par l'épaulière (11) configurée pour entourer l'arrière du cou du porteur ; et
un appuie-tête (14) fixé de manière ajustable à la pièce de tête (12) pour supporter l'arrière de la tête du porteur ; et
un composant de support avant (16) agencé avec un support de menton (19) monté de manière mobile sur celui-ci et fixé à la pièce de tête (12),
**caractérisée en ce que** ledit support de menton (19) est agencé de manière coulissante sur un composant de rotation mobile (22) qui est librement rotatif dans trois plans de trois axes de rotation pour permettre une rotation cervicale tout en empêchant une flexion, une extension et une flexion latérale cervicales excessives du cou.

2. Minerve selon la revendication 1, **caractérisée en ce que** le composant de rotation mobile (22) peut tourner dans un plan horizontal, sagittal et frontal.

3. Minerve selon les revendications 1 et 2, **caractérisée en ce que** le composant de support avant (16) présente une forme ondulée avec au moins un point pivotant supérieur (17).

4. Minerve selon la revendication 3, **caractérisée en ce qu'**un support rotatif (18) est fixé au composant de support avant (16) au niveau de l'au moins un point pivotant supérieur (17).

5. Minerve selon la revendication 4, **caractérisée en ce que** le support rotatif (18) comprend une liaison de support rotatif (21) avec le composant de rotation mobile (22) agencé sur celle-ci, ladite liaison de support rotatif (21) relie le support rotatif (18) au composant de support avant (16).

6. Minerve selon la revendication 5, **caractérisée en ce que** le support de menton (19) est couplé de manière coulissante au composant de rotation mobile (22) au moyen d'un agencement coulissant (23) permettant un mouvement rotatif simultané du support de menton (19) dans un plan horizontal, sagittal, et frontal sur le support rotatif (18).

7. Minerve selon la revendication 6, **caractérisée en ce que** l'agencement coulissant (23) comprend un moyen coulissant (31) qui coulisse dans un moyen de réception d'élément coulissant (32) sensiblement sans friction.

8. Minerve selon l'une quelconque des revendications précédentes 5 à 7, **caractérisée en ce que** le composant de rotation mobile (22) est relié à la liaison de support rotatif (21) au moyen d'une vis.

9. Minerve selon l'une quelconque des revendications précédentes 5 à 8, **caractérisée en ce que** le composant de rotation mobile (22) présente un axe vertical de rotation et permet au support de menton (19) de tourner dans un mouvement de rotation de 20° ou moins dans une quelconque direction autour dudit axe vertical dans le plan horizontal de rotation par rapport au porteur.

10. Minerve selon l'une quelconque des revendications précédentes 5 à 9, **caractérisée en ce que** le composant de rotation mobile (22) présente un axe frontal et permet au support de menton (19) de tourner dans un mouvement de rotation maximal de 10° dans une quelconque direction autour dudit axe frontal dans le plan sagittal de rotation par rapport au porteur.

11. Minerve selon l'une quelconque des revendications précédentes 5 à 10, **caractérisée en ce que** le composant de rotation mobile (22) présente un axe sagittal et permet au support de menton (19) de tourner dans un mouvement de rotation maximal de 10° dans une quelconque direction autour dudit axe sagittal dans le plan frontal de rotation par rapport au porteur.

12. Minerve selon l'une quelconque des revendications précédentes 3 à 11, **caractérisée en ce que** le composant de support avant (16) est fixé de manière ajustable à la pièce de tête (12) pour réguler la distance entre la pièce de tête (12) et le point pivotant supérieur (17) sur le composant de support avant (16) de sorte que le point pivotant supérieur (17) avec le support rotatif (18) coïncide avec le menton du porteur.

13. Minerve selon l'une quelconque des revendications précédentes 6 à 12, **caractérisée en ce que** le mouvement coulissant du moyen coulissant (31) avec le support de menton (19) est restreint par des moyens de régulation ajustables (33).

14. Minerve selon la revendication 13, **caractérisée en ce que** le mouvement coulissant de l'élément coulissant (31) avec le support de menton (19) est totalement restreint.

15. Minerve selon l'une quelconque des revendications précédentes 1 à 14, **caractérisée en ce que** la minerve comprend en outre un dispositif de surveillance électronique agencé pour enregistrer, surveiller et stocker des informations concernant des mouvements coulissants du support de menton (19).
